# EUROPÄISCHE PATENTSCHRIFT

(11) **EP 0 544 274 B1**
(45) Veröffentlichungstag und Bekanntmachung des Hinweises auf die Patenterteilung: **03.03.1999**
(21) Anmeldenummer: 92120128.1
(22) Anmeldetag: 25.11.1992
(51) Int. Cl.: A61B 17/00, A61C 19/00, A61G 15/14

(54) **Ärztliche Behandlungseinrichtung, insbesondere für chirurgische Zwecke, mit einer Versorgungsleitung in Form einer flexiblen Rohrleitung**
Medical treatment device, in particular for surgical purposes with supply conduit having the shape of a flexible tube
Dispositif de traitement médical, en particulier à des fins chirurgicales avec conduit d'alimentation en forme de tube flexible

(30) Priorität: 25.11.1991 DE 4138672
(43) Veröffentlichungstag der Anmeldung: 02.06.1993
(73) Patentinhaber: KALTENBACH & VOIGT GMBH & CO., 88400 Biberach (DE)
(72) Erfinder: Strohmaier, Ernst, W-7953 Bad Schussenried (DE)
(74) Vertreter: Schmidt-Evers, Jürgen, Dipl.-Ing.

(56) Entgegenhaltungen:
- EP-A- 0 024 255
- EP-A- 0 317 521
- WO-A-88/02621
- BE-A- 550 488
- FR-A- 2 340 714

## Beschreibung

Die Erfindung bezieht sich auf eine Behandlungseinrichtung nach dem Oberbegriff des Anspruchs 1 und ein Verfahren nach dem Oberbegriff des Anspruchs 9..

Bei der Behandlung des menschlichen oder tierischen Körpers mit einem ärztlichen Behandlungsinstrument ist in vielen Fällen die Zugabe eines gasförmigen oder insbesondere flüssigen Behandlungsmittels erforderlich, wobei es sich hierbei um Luft, Wasser oder ein Gemisch davon (Spray) handeln kann. In vielen Fällen, insbesondere in der chirurgischen und mikrochirurgischen Behandlung ist eine Kühlflüssigkeit erforderlich, wobei es üblich ist, eine Salzlösung (NaCl) zu verwenden.

Das Behandlungsmittel wird dem Behandlungsinstrument durch eine flexible Leitung, insbesondere eine Schlauchleitung, zugeführt. Es ist bereits vorgeschlagen worden, die Schlauchleitung am oder in der Versorgungsleitung anzuordnen.

Insbesondere dann, wenn als Behandlungs- bzw. Kühlflüssigkeit eine mineralische Lösung, wie z.B. eine Salzlösung, verwendet wird, dann besteht die Gefahr von Kristallisation und Ablagerungen in der flexiblen Rohrleitung, die deren freien Querschnitt verringern oder sogar verstopfen und somit die Behandlung bzw. Kühlung beeinträchtigen können. Diese Gefahr ist umso größer je kleiner der Querschnitt der Schlauchleitung ist.

Es ist auch bereits vorgeschlagen worden, die flexible Rohrleitung im dem Behandlungsinstrument abgewandten Endbereich der Versorgungsleitung etwa radial aus diesem herauszuführen und somit unter Umgehung der Steckverbindung für die Versorgungsleitung an die vorhandene Behandlungsmittelquelle bzw. Pumpe anschließen zu können.

In der DE-A- 024 255 ist eine ärztliche Behandlungseinrichtung und ein Verfahren nach dem Oberbegriff des Anspruchs 1 bzw. 9 mit einem Verteiler für Fluide unter Druck beschrieben. Bei diesem bekannten Stand der Technik sind mehrere Vorratsbehälter für Behandlungsflüssigkeiten vorgesehen, die jeweils zum einen mittels einer ansteuerbaren Leitungsverbindung mit einer Druckluftquelle und zum anderen jeweils mit einer zu einem Schnellverschluß führenden Abgabeleitung verbunden ist. Des weiteren ist eine direkt mit der Druckluftquelle verbundene Abgabeleitung mit einem Schnellverschluß vorgesehen. Ein Behandlungsinstrument ist mittels einer flexiblen Versorgungsleitung wahlweise an die vorhandenen Schnellverschlüsse anschließbar zwecks Abgabe der Behandlungsflüssigkeiten oder der Druckluft durch das Behandlungsinstrument.

Der Erfindung liegt die Aufgabe zugrunde, eine Behandlungseinrichtung nach dem Oberbegriff des Anspruchs 1 oder ein Verfahren nach dem Oberbegriff des Anspruchs 9 so auszugestalten, daß eine Beeinträchtigung der Flüssigkeitszuführung zur Behandlungsstelle in einfacher und sauberer Weise verhinderbar ist bzw. ausgeräumt werden kann.

Diese Aufgabe wird durch Merkmale des Anspruchs 1 bzw. 9 gelöst. Vorteilhafte Weiterbildung der Erfindung sind in den Unteransprüchen beschrieben.

Der erfindungsgemäßen Behandlungseinrichtung ist eine Spüleinrichtung mit einem Spülflüssigkeits-Vorratsbehälter und einem Sammelbehälter für verbrauchte Spülflüssigkeit zugeordnet, die an die sich in oder an der Versorgungsleitung erstreckenden FlüssigkeitsZuführungsleitung anschiießbar und von dieser wieder trennbar ist, um die Zuführungsleitung bei Bedarf oder in regelmäßigen oder unregelmäßigen Abständen zu spülen. Dabei ist es im Rahmen der Erfindung möglich, der Spüleinrichtung eine eigene Pumpe zuzuordnen oder die vorhandene Pumpe für die Flüssigkeitszuführung auch zum Fördern des Spülmitteis zu benutzen. Im letzteren Fall ist es möglich, die vorhandene Pumpe durch Umschaltventile, die manuell oder auch automatisch betätigbar sind, oder durch Umstecken einer oder mehrerer Leitungsabschnitte an die Spüleinrichtung bzw. deren Vorratsbehälter anzuschließen. Es ist besonders vorteilhaft, die Spülvorgänge zeitabhängig automatisch zu steuern, so daß die behandelnde Person ihre Aufmerksamkeit nicht auf das Spülen zu richten braucht.

Mit dem erfindungsgemäßen Verfahren läßt sich einer unerwünschten Ansammlung von Ablagerungen in der Zuführungsleitung wirksam vorbeugen, oder es lassen sich auch Ablagerungen frei- und ausspülen. Dies kann an Ort und Stelle in der Behandlungseinrichtung ggfs. automatisch erfolgen, d. h., die Zuführungsleitung braucht nicht demontiert zu werden. Um zu gewährleisten, daß bei einer späteren Behandlung am vorhandenen Behandlungsplatz kein Spülmittel-Schub die Behandlungsstelle erreicht wird, wird die Zuführungsleitung nach dem Spülvorgang durch Durchfördern von Luft entleert.

Die in den Unteransprüchen enthaltenen Merkmale tragen zur Problemlösung bei und führen außerdem zu einfachen, praktischen und kostengünstig herstellbaren Ausgestaltungen, die eine sichere Funktion sowie eine einfache Montage oder Demontage und auch Handhabungsfreundlichkeit gewährleisten.

Nachfolgend werden die Erfindung und weitere durch sie erzielbare Vorteile anhand eines bevorzugten Ausführungsbeispiels und einer Zeichnung näher erläutert. Es zeigt
- Fig. 1: eine erfindungsgemäße Behandlungseinrichtung für chirurgische Zwecke in perspektivischer Darstellung von vorne;
- Fig. 2: die in Fig. 1 mit X gekennzeichnete Einzelheit in vergrößerter Darstellung;
- Fig. 3: die Einzelheit in einer anderen Funktionsstellung;
- Fig. 4: die Einzelheit bei abgenommenem Spülmittelbehälterteil;
- Fig. 5: das Behälterteil der Behandlungseinrichtung als Einzelteil in perspektivischer Darstellung;
- Fig. 6: ein Funktionsdiagramm für das Spülen.

Die Hauptteile der Behandlungseinrichtung 1 sind ein Behandlungsinstrument in Form eines Handstücks 2 und ein Steuergerät 3 mit Steuertasten an seiner Frontseite und mit einer integrierten elektronischen Steuereinrichtung. mit der das Handstück 2 durch eine biegsame bzw. flexible Versorgungsleitung 4 verbunden ist. Das Handstück 2 trägt an seinem vorderen Ende ein Behandlungswerkzeug 5, das vorzugsweise gegen wahlweise erforderliche unterschiedliche Werkzeuge austauschbar ist. Bei solchen Behandlungsinstrumenten, bei denen die Zuführung eines Kühlmittels, insbesondere einer Kühlflüssigkeit wie eine Salzlösung (NaCl), zur Behandlungsstelle erforderlich ist, ist dem Steuergerät 3 eine Kühlmittelpumpe 6 zugeordnet, die vorzugsweise am Steuergerät 3 angebaut oder in diese integriert sein kann. Die zum vorderen Ende des Handstücks 2 führende Kühlmittelleitung 7 verläuft wenigstens teilweise durch die Versorgungsleitung 4 bzw. das Handstück 2. Am Steuergerät 3 ist eine Steckkupplung 8 angeordnet, in die ein am freien Ende der Versorgungsleitung 4 angeordneter Stecker 9 einsteckbar ist zwecks Anschluß an die elektronische Steuereinrichtung. Das Steuergerät 3 kann auf einem Gestell oder Tisch 11 stehen. Es ist eine Haltevorrichtung mit einem Ablageteil für das Handstück 2 in dessen Bereitschaftsstellung vorzugsweise am Steuergerät 3 angeordnet. Bei der vorliegenden Ausgestaltung ist ein Ablageteil in Form einer muldenförmigen, schräg zum Behandlungsplatz hin geneigten Schale oder Platte 13 vorgesehen, auf der das Handstück 2 in einer mit dem Werkzeug 5 schräg nach unten weisenden Position formschlüssig aufliegt. Das Handstück 2 ist aufgrund der muldenförmigen Form der Platte 13 gegen seitliches Verlagern gesichert. Vorzugsweise ist der Muldengrund an die taillierte Form des Handstücks 2 angepaßt, so daß sich auch in dessen Längsrichtung eine formschlüssige Halterung ergibt. Die Platte 13 ist an einem etwa horizontalen, vom Tisch 11 oder vom Gehäuse des Steuergeräts 3 wegragenden Tragarm 12a gehalten, an dessen Ende sie vorzugsweise in einem Gelenk um eine etwa vertikale Schwenkachse frei schwenkbar gelagert ist.

Der Tisch 11 steht mit Rollen (nicht dargestellt) auf dem Boden und ist durch manuellen Zugriff, vorzugsweise an einem frontseitigen Handgriff 11a verschiebbar. Der Tisch 11 ruht auf dem oberen Ende einer Tragsäule 11b, die auf dem Stegteil 10a eines U-förmigen Standrahmens 10 steht, dessen Schenkelteile 10b zur Bedienungsseite hin weisen.

Der Tragarm 12a ist längenveränderlich und an seinem der Haltevorrichtung abgewandten Ende in einem Gelenk 14 mit vertikaler Schwenkachse 15 mit dem Steuergerät 3 verbunden. Aufgrund dieser Ausgestaltung ist die Haltevorrichtung mit dem Handstück 2 sowohl horizontal frei schwenkbar als auch bezüglich ihres Abstandes zum Steuergerät 3 einstellbar. Hierdurch ist sie in einem großen Bewegungsbereich frei verstellbar und in bezüglich des Behandlungsplatzes oder der Behandlungsstelle günstigen, wahlweisen Bereitschaftsstellungen einstellbar, in denen die behandelnde Person das Handstück handhabungsfreundlich ergreifen und wieder ablegen kann. In der Zeichnung ist der Tragarm 12a mit gestrichelten Linien in seiner ausgezogenen Position dargestellt. Bei dieser andeutungsweisen Darstellung fehlt die Haltevorrichtung, um das sie tragende Gelenk 16 am freien Ende des Tragarms 12a zu zeigen, das durch eine vertikale Bohrung gebildet sein kann. in der ein von der Haltevorrichtung nach unten ragender runder Zapfen (nicht dargestellt) von oben mit Bewegungsspiel einsteckbar ist.

Im Rahmen der Erfindung kann es sich bei dem Tragarm 12a um einen teleskopierbaren Tragarm handeln. Bei der vorliegenden Ausgestaltung ist der Tragarm 12a im Sinne eines Kniehebels mit zwei Tragarmteilen 12b, 12c ausgebildet, die an ihren einander zugewandten Enden in einem mittleren Gelenk 18 mit vertikaler Schwenkachse miteinander verbunden sind. Vorzugsweise liegen die beiden miteinander verbundenen Enden der Tragarmteile 12b, 12c aufeinander, wobei sie sich überlappen und von einem Gelenkbolzen in vertikalen Bohrungen durchsetzt sind. Es ist zum einen wegen Raumersparnis und zum anderen aus Gründen hinreichende Stabilität von Vorteil, die Tragarmteile 12b 12c aus flachen Leisten rechteckigen Querschnitts zu bilden, die flach angeordnet sind.

Bei der vorliegenden Ausgestaltung ist im unteren Bereich des kastenformigen Steuergeräts, hier unterhalb der Bedienungs- bzw. Tastaturfläche, eine horizontale Ausnehmung 21 vorgesehen, in die der Tragarm 12a hineinschwenkbar ist. Dabei kann das Gelenk 14 im Bereich der Frontseite des Steuergeräts 3 oder innerhalb der Ausnehmung 21 angeordnet sein, wie es in der Zeichnung dargestellt ist. Die Ausnehmung 21 ist bei der vorliegenden Ausgestaltung im vorderen linken Eckenbereich des Steuergeräts 3 angeordnet, und sie ist sowohl zur Bedienungsseite d.h. von vorne und von der linken Seite zugänglich bzw. offen. Die sich von der linken Seite des Steuergeräts 3 nach rechts erstreckende Breite b der Ausnehmung 21 entspricht in etwa einem dreiviertel der Breite B des Steuergeräts 3, so daß die Ausnehmung 21 in einem Abstand von der rechten Seite des Steuergeräts 3 endet. In der eingeschwenkten Stellung befindet sich die Platte 13 unmittelbar vor der rechten Frontseitenhälfte des Steuergeräts 3. Die Höhe der Ausnehmung 21 ist so groß bemessen, daß der Tragarm 12a mit vertikalem Bewegungsspiel einschwenkbar ist.

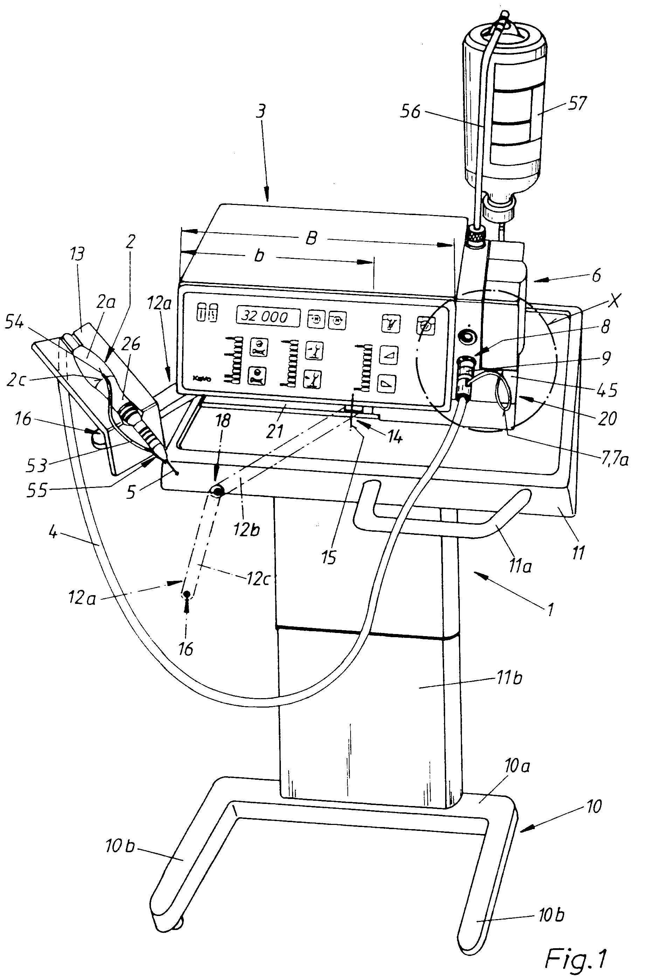

Vorzugsweise sind die Breite b der Ausnehmung 21 und die Länge des inneren Tragarmteils 12b so aneinander angepaßt, daß in der eingeschwenkten Stellung gem. Figur 1 das innere Tragarmteil 12b bzw das mittlere Gelenk 18 nicht seitlich aus der Ausnehmung 21 herausragen.

Vorzugsweise ist die Ausnehmung 21 in einem insbesondere plattenförmigen Untersatz, hier in einer Grundplatte 3a des kastenförmigen Steuergerätes 3 ausgebildet. Die Grundplatte 3a ist lösbar am Steuergerät 3 befestigbar z.B. durch Schrauben. Die Vorrichtung bzw. das Steuergerät 3 ist somit durch die ansetzbare Grundplatte 3a ggfs. mit der Haltevorrichtung modular ergänzbar oder wahlweise nachrüstbar.

Die vorbeschriebene Anordnung und Ausgestaltung der Haltevorrichtung ermöglicht eine wahlweise Einstellung der Bereitschaftsstellung des Handstücks 2 im gesamten Frontbereich des Steuergerätes 3. Die auszuwählende Bereitschaftsstellung der Haltevorrichtung kann somit an eine Vielzahl und auch schwierige Behandlungspositionen angepaßt werden.

Bei der vorliegenden Ausgestaltung ist die Pumpe 6 durch eine Schlauchpumpe gebildet, die im etwa kastenförmigen Gehäuse 22 des Steuergeräts 3 angeordnet ist. Bei den die Schlauchpumpe 6 aufnehmenden Gehäuseteil kann es sich auch um ein Anbaugehäuse handeln, das das Gehäuse 22 ergänzt. Bei der vorliegenden Ausgestaltung ist die Schlauchpumpe 6 im rechten Endbereich des Gehäuses 22 in etwa mittlerer Höhe angeordnet, wobei die Drehachse 23 der Schlauchpume 6 horizontal und parallel zur Bedienungsseite 24 des Steuergeräts 3 verläuft und in der Seitenansicht von rechts die Schlauchpumpe 6 etwa mittig im Gehäuse 22 angeordnet ist.

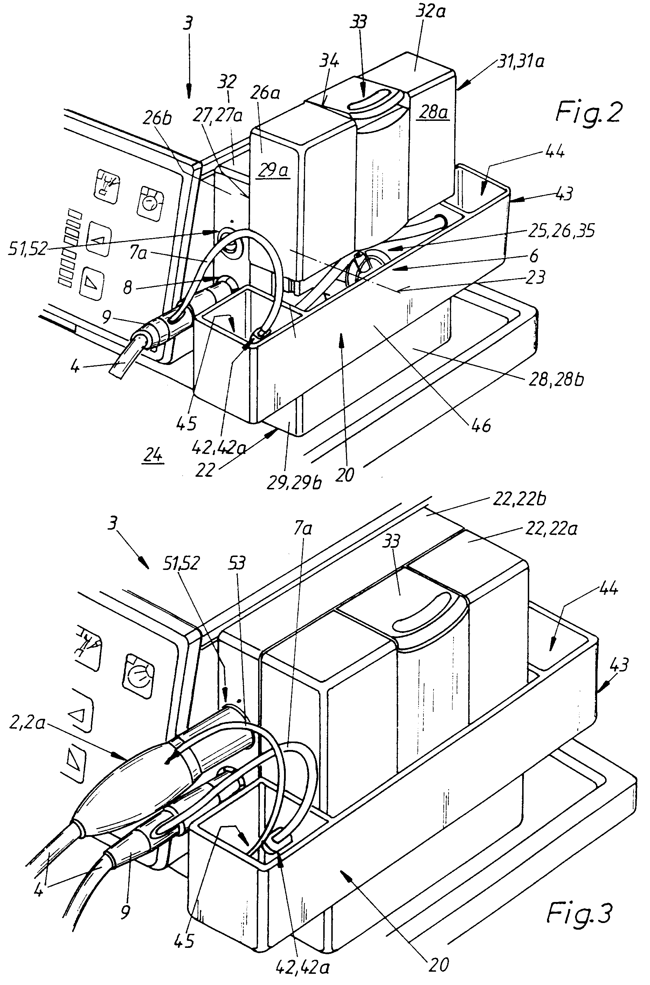

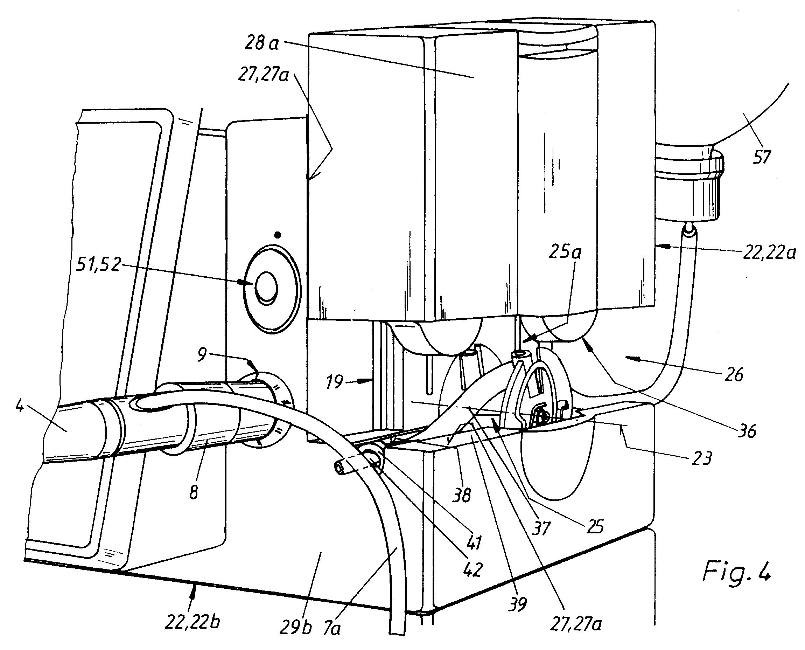

Die Zugänglichkeit zum Pumpenraum 25 der Schlauchpumpe 6 ist durch eine Gehäuseöffnung 26 gewährleistet, die durch die Verschiebung nach oben eines Gehäuseteils 22a zu öffnen und durch dessen Verschiebung nach unten wieder zu schließen ist. Das Gehäuseteil 22a ist durch eine winkelförmige Teilungsfuge 27 mit ebenen Teilungsfugenabschnitten 27a, 27b vom übrigen Gehäuseteil 26b getrennt, wobei der Teilungsfugenabschnitt 27a sich vorzugsweise vertikal erstreckt nach oben ausläuft und der Teilungsfugenabschnitt 27b etwa in der Höhe der Drehachse 23 vom ersten Teilungsfugenabschnitt 27a horizontal verläuft und in der zugehörigen, hier rechten Seitenwand 28 ausläuft. Von vorne, d.h. von der Bedienungsseite 24 aus gesehen, erstrecken sich die Teilungfugenabschnitte durchgehend durch das Gehäuse von dessen Vorderwand 29 bis zu dessen Rückwand 31. Das Gehäuseteil 22a befindet sich somit im oberen rechten Eckenbereich des Gehäuses 22, und es umfaßt ein Seitenwandteil 28a, ein Vorderwandteil 29a, ein Deckwandteil 32a der Deckwand 32 des Gehäues 22 und ein Rückwandteil 31a. Das Gehäuseteil 22a ist in einer nur in Fig. 4 dargestellten, zwischen ihm und dem übrigen, stationären Gehauseteil 22b angeordneten Führung 19 zwischen der in Figur 2 dargestellten Offenstellung und der in Fig.3 dargestellten Schließstellung vorzugsweise vertikal verschiebbar und in der jeweiligen Endstellung positionierbar. Vorzugsweise ist das Gehäuseteil 22a durch eine Feder in seine obere Offenstellung vorgespannt und in seiner unteren Schließstellung durch eine integrierte, lösbares Verriegelungsvorrichtung einrastbar. Hierzu dient bei der vorliegenden Ausgestaltung eine mit der Oberseite des Deckwandteils 32a abschließende Drucktaste 33, die in einer im oberen Bereich des Gehäuseteils 22a angeordneten Ausnehmung 34 die Gehäusekontur in etwa ersetzend angeordnet und um eine horizontale, rechtwinklig zur Drehachse 23 verlaufende Schwenkachse schwenkbar, nämlich zur rechten Seite hin kippbar in einem in das Gehäuseteil 22a integrierten Gelenk gelagert ist. Die vorbeschriebene Verriegelungsvorrichtung wird durch manuellen Druck auf die Drucktaste 33 gelöst, wobei die Drucktaste 33 zur Seite schwenkt bzw. kippt. Zum Schiießen ist das Gehauseteil 22a manuell herunterzudrücken, bis die Verriegelvorrichtung in der Schließstellung selbsttätig einrastet.

Die Pumpenteile der Schlauchpumpe 6 sind von an sich bekannter Art und sie werden durch einen von einem nicht dargestellten Motor antreibbaren und mit einander diametral gegenüberliegenden Quetschrollen und seitlichen Führungsrollen bestückten Rotor 35 und einem mit diesem zusammenwirkenden Widerlager 36 (Fig. 4) gebildet, das mit gabelförmigen Widerlageransätzen wellenförmig ausgebildet ist und den Rotor 35 von oben seitlich übergreift. Die den vertikalen Teilungsfugenabschnitt 27a enthaltende Vertikalebene befindet sich in etwa am inneren Ende des Rotors 35. Die den horizontalen Teilungsfugenabschnitt 27b enthaltende Horizontalebene liegt in etwa in Höhe der Drehachse 23 (Fig. 4). Der Pumpenschlauch 37 der Schlauchpumpe läßt sich bei geöffnetem Gehäuseteil 22a leicht und bequem von der Seite her in den sich zwischen dem Rotor 35 und dem Widerlager 36 befindlichen ebenfalls geöffneten Arbeitsraum 25a einlegen, so daß er sich bogenförmig über den Rotor 35 erstreckt. Die Endbereiche des Pumpenschlauchs 37 sind in quer zur Drehachse 23 verlaufenden Schlitzen 38 in den Teilungsfugenabschnitt 27b versenkt, die sich in oberseitig mit dem Teilungsfugenabschnitt 27b abschließenden Wandteilen 39 des stationären Gehäuseteils 28b befinden. In den Seitenwandteilen 29b des stationären Gehäuseteils 22b sind mit den Schlitzen 38 fluchtende, nach oben auslaufende Durchführungslöcher 41 für den Pumpenschlauch 37 vorhanden, wobei im vorderseitigen Durchführungsloch 41 eine mit dem Pumpenschlauch 37 verbundene Anschlußarmatur 42 von oben eingesetzt und in Längsrichtung des Pumpenschlauchs 37 durch Eingriff in eine Nut des Durchführungslochs 41 in Längsrichtung des Pumpenschlauchs 37 unverschiebbar formschlüssig gehalten ist.

Ein in der Fig. 4 zwecks Verbesserung der Übersicht nicht dargestelltes Spülmittelbehälterteil 43 weist einen Spülmittelvorratsbehälter 44 und einen Sammelbehälter 45 für verbrauchtes Spülmittel auf. Diese Behälter 44, 45 sind an einer vertikalen Verbindungswand 46, vorzugsweise einstückig, ausgebildet, so daß sie zu einer Breitseite dieser Verbindungswand 46 vorspringen. Der Abstand der Behälter 44, 45 voneinander ist an die sich quer zur Bedienungsseite 24 erstreckende Breite des Gehäuses 22 angepaßt, so daß das Spülmittelbehälterteil 43 von der Seite her horizontal so an das rechte Stirnende des Gehäuses 22 ansetzbar ist, daß die Behälter 44, 45 das Gehäuse 22 vorderseitig bzw. rückseitig übergreifen. Die sich parallel zur Bedienungsseite 24 erstreckende Breite der Behälter 44, 45 ist vorzugsweise an die entsprechende Breite des beweglichen Gehäuseteils 22a angepaßt. Zur Halterung des Spülmittelbehälterteils 43 in dieser angesetzten Position, in der es den horizontalen Teilungsfugenabschnitt 27a etwas nach oben überragt, ist das Spülmittelbehälterteil 43 am stationären Gehäuseteil 22b durch nicht dargestellte Mittel, z.B. es untergreifende Ansätze am stationären Gehäuseteil 22b, lösbar gehalten bzw. befestigt. Dabei durchsetzt die Anschlußarmatur 42 auch die ihr gegenüberliegende Innenwand 47 des Sammelbehälters 45 in einem Loch bei Gewährleistung der Abdichtung mittels eines Dichtungsrings oder dergleichen. Die Anschlußarmatur 42 kann auch nur in der Innenwand 47 im Sammelbehälter 45 sitzen. z.B. eingeschraubt sein, vorzugsweise in Form einer Winkel-Armatur 42a. In der gegenüberliegenden Innenwand 47a ist fluchtend mit dem hinteren Durchführungsloch 41 ein Loch 50 für den Pumpenschlauch 37 vorgesehen. Der Anschluß an den aus der Versorgungsleitung 4 radial herausgeführten Schlauch 7a ist durch dessen Verbindung mit der Anschlußarmatur 42 bzw. 42a geschaffen. Der Pumpenschlauch 37 ist im Loch 50 abgedichtet oder das Loch 50 befindet sich oberhalb des Füllpegels im Vorratsbehälter 44.

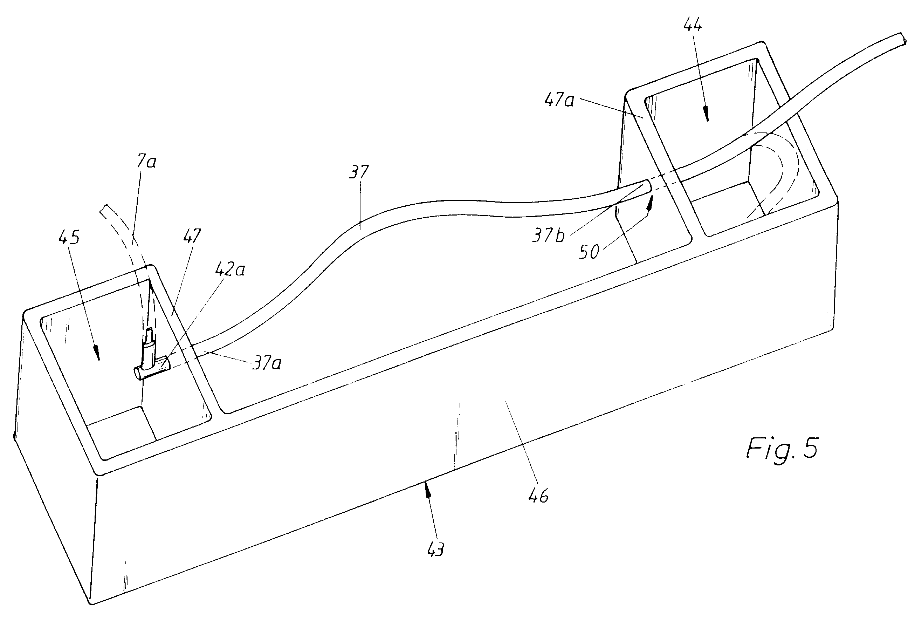

Fig. 5 zeigt das Spülmittelbehälterteil 43 als lösbar angeordnetes Anbauteil, das dadurch am Gehäuse 22b im Sinne einer Steckfassung gehalten ist, daß die innenseitig an den Innenwänden 47, 47a vorhandenen Pumpenschlauchabschnitte 37a, 37b in die Durchführungslöcher 41 einfassen. Das Behälterteil 43 kann somit durch Einhängen leicht demontiert bzw. montiert werden, z.B. zum Entleeren des Sammelbehälters 45. Bei einem separaten und wahlweise anbaubaren Spülmittelbehälterteil 43 ist letzterem vorzugsweise ein eigener Pumpenschlauch zugeordnet, so daß ein Verbinden des Pumpenschlauchs mit der Anschlußarmatur 42a und ein Einfädeln im Loch 50 entfällt. Bei einer solchen Ausgestaltung ist vor dem Ansetzen des Spülmittelbehälters 43 mit dem eigenen Pumpenschlauch der Pumpenschlauch 37 zu entfernen.

Der Anschluß an den aus der Versorgungsleitung 4 radial herausgeführten Schlauch 7a ist durch seinen Anschluß an die Anschlußarmatur geschaffen.

In der Nähe des vorne am Gehäuse 22 angeordneten Sammelbehälters 45, vorzugsweise über der links daneben angeordneten Steckkupplung S, ist am Steuergerät 3 bzw. an dessen Gehäuse 22 eine Parkstelle in Form einer Halteeinrichtung 51 für das Handstück 2 vorgesehen. Die Halteeinrichtung 51 kann durch eine Steckvorrichtung gebildet sein, in die das Handstück 2 einlegbar oder einsteckbar ist. Bei der vorliegenden Ausgestaltung ist die Halteeinrichtung 51 durch ein horizontales Loch 52 an der Vorderseite des Steuergeräts 3 gebildet, in das das Handstück 2 bzw. ein Teil desselben einsteckbar ist. Bei der vorliegenden Ausgestaltung besteht das Handstück aus zwei hintereinander angeordneten und durch eine Schnellkupplung bzw. Steckfassung miteinander verbindbaren Handstückteilen, nämlich einem hinteren, mit der Versorgungsleitung 4 verbundenen Handstückteil 2a und einem vorderen, das Behandlungswerkzeug 5 tragenden Handstückteil 2b. Dabei ist die Anordnung so getroffen, daß das hintere Handstückteil 2a nach seiner Trennung vom vorderen Handstückteil 2b in das Loch 52 einsteckbar ist.

Beim vorliegenden zweistückigen Handstück 2 überbrückt die Kühlmittelleitung 7 die Teilungsfuge 2c des Handstücks 2 außenseitig. Hierzu ist ein Schlauchabschnitt 53 vorgesehen, der mit seinen Enden an Anschlußarmaturen 54, 55 angeschlossen ist, die von der zugehörigen Kühlmittelleitung 7 ausgehend am hinteren Handstückteil 2a und vorderen Handstückteil 2b angeordnet sind, Der Abstand der Parkstelle bzw. Halteeinrichtung 51 vom Sammelbehälter 45 ist nur so groß bzw. der Schlauchabschnitt ist so lang, daß in der in der Halteeinrichtung 51 aufgenommenen Stellung des Handstücks 2 der Schlauchabschnitt 53 in den Sammelbehälter 45 eingehängt werden kann. In dieser Position kann ein Spülvorgang erfolgen. Sofern die Anordnung vorher für die Zuführung von Kühlmittel eingerichtet war, ist der Pumpenschlauch 37 vom vorhandenen, an einer Trägerstange 56 am Steuergerät 3 aufgehängten, verschließbaren Kühlmittelvorratsbehälter 57 durch lösen der vorhandenen Anschlußverbindung zu trennen und mit seinem freien Ende in den Spülmittelvorratsbehälter 44 einzustecken. Dann kann der Spülvorgang mit der Spüleinrichtung 20 stattfinden, der z.B. durch Betätigung eines Betätigungselements an der vorhandenen Steuertastatur eingeleitet wird. Vorzugsweise ist dem vorhandenen Steuergerät 3 eine Abschaltvorrichtung für den Spülvorgang zugeordnet, die ihn nach einer vorbestimmbaren Zeit automatisch abschaltet. Die dabei von der Schlauchpumpe 6 aus dem Spülmittelvorratsbehälter 44 gesaugte und weiter geförderte Spülmittelmenge sollte vorzugsweise etwa dem zwei- bis dreifachen Volumen des Pumpenschlauchs 37 oder der Spülmittelleitung insgesamt entsprechen. Das durchgespülte Spülmittel, vorzugsweise Wasser, wird mittels des Schlauchabschnitts 53 in den Sammelbehälter 45 eingeführt. Beim Durchspülen werden in der Kühlmittelleitung 7 vorhandene Ablagerungen oder Kristalle der Salzlösung frei- und weggespült. Hierdurch wird eine Beeinträchtigung des freien Durchflußquerschnitts der Kühlmittelleitung 7 und somit auch eine Beeinträchtigung der Kühlung verhindert.

Es ist auch möglich, die Haltevorrichtung zum Halten des Handstücks 2 in der Parkstellung zu benutzen. Dies ist möglich, weil die Haltevorrichtung aufgrund der horizontalen Beweglichkeit des Haltearms 12a bis nahe an den Sammelbehälter 45 schwenkbar ist. Bei einer Benutzung der Haltevorrichtung braucht das Handstück 2 nicht demontiert zu werden, wenn diese Haltevorrichtung so weit an dem Sammelbehälter 45 schwenkbar ist, daß das freie Ende des Handstücks 2 mit dein vorhandenen Kühlmittelausgang in den Sammelbehälter 45 ragt.

Nach Beendigung des Spülvorgangs ist der Pumpenschlauch 37c mit seinem hinteren Ende wieder an den Kühlmittelvorratsbehäler 57 anzuschließen.

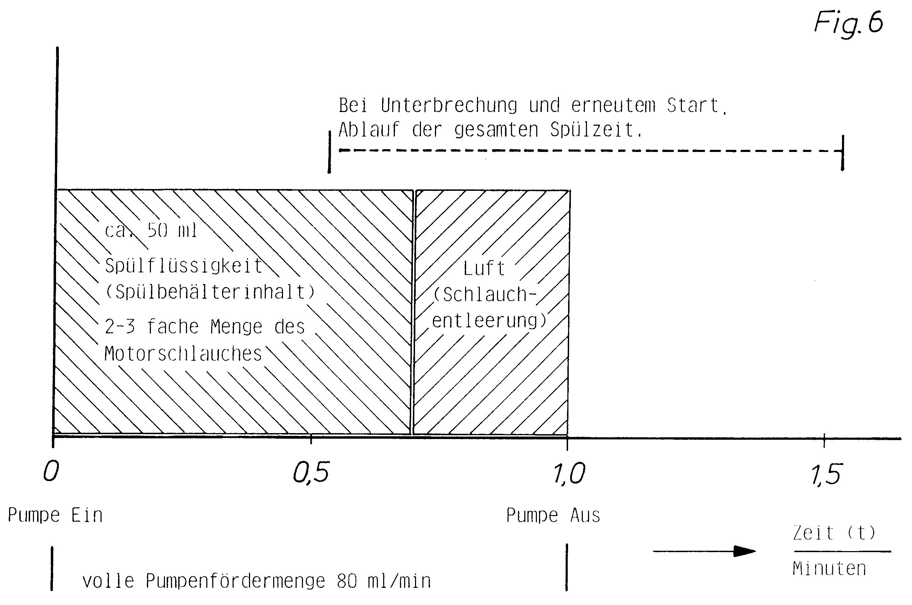

Wie die einen Spülmittelzyklus verdeutlichende Fig. 6 zeigt, wird vozugsweise am Ende des Spülvorgangs nur Luft gefördert, um das Spülmittel aus der Zuführungsleitung 7 zu verdrängen. Das Luftvolumen beträgt vorzugsweise etwa 1/5 bis 1/2, insbesendere etwa 1/3 des Spülmittelvolumens, ist jedoch größer als das Volumen in der Zuführungsleitung 7, um das darin befindliche Spülmittel verdrängen zu können. Zur Beendigung der Spülmittelförderung ist deshalb der Pumpenschlauch 37 aus dem Vorratsbehälter 44 herauszunehmen, oder der Vorratsbehälter 44 ist so groß bemessen, bzw. so zu befüllen, daß der Pumpenschlauch 37 nach der Spülmittelförderung Luft saugt. Bei einer Steuerung mit Ventilen ist eine Luftansaugleitung zu verbinden bzw. zu öffen.

Bei der vorliegenden Ausgestaltung beträgt der Spülvorgang insgesamt etwa 1 min. Die vorhandene Steuerung ist vorzugsweise so ausgelegt, daß bei einer Unterbrechung des Spülvorgangs letzterer erneut eingeschaltet wird und im vollen Umfang, nämlich Spülmittelförderung und Luftförderung, abläuft.

Die Erfindung umfaßt auch ein vorteilhaftes Spülverfahren.

## Patentansprüche

1. Ärztliche Behandlungseinrichtung (1), bestehend aus:
- einem Behandlungsinstrument in Form eines Handstücks (2), das durch eine Versorgungsleitung (4) mit einem Steuergerät (3) verbunden ist,
- wobei das Handstück (2) aus einem vorderen und einem hinteren Handstückteil (2a, 2b) besteht,
- einer Medienquelle für eine mineralische Behandlungslösung als Behandlungsmittel, an die die Versorgungsleitung (4) mit einer sich an sie oder durch sie erstreckenden flexiblen Leitung (7, 7a) angeschlossen ist,
- einer Spüleinrichtung (20) mit einer Spülmittelquelle,
- und einer Halteeinrichtung (51) für das Handstück (2) am Steuergerät (3),
**dadurch gekennzeichnet,**
- daß die Spüleinrichtung (20) einen Sammelbehälter (45) für verbrauchtes Spülmittel besitzt,
- die Handstückteile (2a, 2b) durch eine Schnellkupplung miteinander verbindbar sind,
- die Leitung (7) einen die Teilungsfuge (2c) zwischen den Handstückteilen überbrückenden Schlauchabschnitt (53) aufweist, der durch Anschlußarmaturen (54, 55) mit den Handstückteilen (2a, 2b) lösbar verbunden ist
- und die Halteeinrichtung (51) als Aufnahmeloch (52) zur Aufnahme des hinteren Handstückteils (2a) ausgebildet ist und so in der Nähe des Sammelbehälters (45) angeordnet ist, daß der Schlauchabschnitt (53) in den Sammelbehälter (45) eingehängt werden kann.

2. Behandlungseinrichtung nach Anspruch 1,
**dadurch gekennzeichnet,**
daß die Spülmitteleinrichtung (20) automatisch durch Betätigung elektrischer Steuerelemente oder manuell an die flexible Leitung (7, 7a) anschließbar ist.

3. Behandlungseinrichtung nach Anspruch 1 oder 2,
**dadurch gekennzeichnet,**
daß die Spülmitteleinrichtung (20) einen Spülmittelvorratsbehälter (44) und eine Spülmittelpumpe (6), insbesondere eine Schlauchpumpe, aufweist.

4. Behandlungseinrichtung nach Anspruch 3,
**dadurch gekennzeichnet,**
daß die Spülmittelpumpe (6) eine gemeinsame Pumpe (6) für die Zuführung (13) des Behandlungsmittels und des Spülmittels darstellt.

5. Behandlungseinrichtung nach wenigstens einem der vorherigen Ansprüche,
**dadurch gekennzeichnet,**
daß der Sammelbehälter (45) im vorderen Bereich des Steuergeräts (3) angeordnet ist.

6. Behandlungseinrichtung nach wenigstens einem der vorherigen Ansprüche,
**dadurch gekennzeichnet,**
daß das Aufnahmeloch (52) am Steuergerät (3) angeordnet ist.

7. Behandlungseinrichtung nach Anspruch 6,
**dadurch gekennzeichnet,**
daß das Aufnahmeloch (52) an der Frontseite des Steuergeräts (3) angeordnet ist.

8. Behandlungseinrichtung nach Wenigstens einem der vorherigen Ansprüche,
**dadurch gekennzeichnet,**
daß ein Behälterteil (43) mit einem Vorratsbehälter (44) für das Spülmittel und dem Sammelbehälter (45) vorgesehen ist und daß eine als flexibler Pumpenschlauch (37) ausgebildete Förderleitung wahlweise mit einem Vorratsbehälter (57) für das Behandlungsmittel oder mit dem Vorratsbehälter (44) für das Spülmittel verbindbar ist.

9. Verfahren zum Reinigen der flexiblen Leitung (7, 7a), der ärztlichen Behandlungseinrichtung (1) gemäß Anspruch 1, wobei die Leitung (7, 7a) als Rohrleitung zum Zuführen einer mineralischen Behandlungsflüssigkeit von der Medienquelle (6) zum Behandlungsinstrument in Form eines Handstücks (2) ausgebildet ist, bei dem nach einer Behandlung oder nach bestimmten oder bestimmbaren Zeitabschnitten ein flüssiges Spülmittel und dann ein gasförmiges Medium durch die Leitung (7, 7a) gefördert und das flüssige Spülmittel mit dem gasförmigen Medium aus der Rohrleitung (7, 7a) entleert wird.

## Claims

1. Medical treatment device (1), comprising:
- a treatment instrument in the form of a handpiece (2) which is connected with a control apparatus (3) by means of a supply line (4),
- the handpiece (2) comprising a forward and a rearward handpiece part (2a, 2b),
- a media source for a mineral treatment solution as a treatment agent, to which the supply line (4) is connected with a flexible line (7, 7a) extending thereon or therethrough,
- a flushing device (20) having a flushing agent source,
- a holder device (51) for the handpiece (2) at the control apparatus (3),
characterised in that,
- the flushing device (20) has a collection container (45) for used flushing agent,
- the handpiece parts (2, 2a) can be connected with one another by means of a quick coupling,
- the line (7) has a hose section (53) bridging the dividing joint (2c) between the handpiece parts, which section is releasibly connected with the handpiece parts (2a, 2b) by means of connection fittings (54, 55),
- and the holder device (51) is formed as a receiving hole (52) for receiving the rearward handpiece part (2a) and is so arranged in the vicinity of the collection container (45) that the hose section (53) can be suspended in the collection container (45).

2. Treatment device according to claim 1,
characterised in that,
the flushing agent device (20) is connectible to the flexible line (7, 7a) automatically by operation of electrical control elements, or manually.

3. Treatment device according to claim 1 or 2,
characterised in that,
the flushing agent device (2) has a flushing agent supply container (44) and a flushing agent pump (6), in particular a hose pump.

4. Treatment device according to claim 3,
characterised in that,
the flushing agent pump (6) is a common pump (6) for the delivery (13) of the treatment agent and the flushing agent.

5. Treatment device according to any preceding claim,
characterised in that,
the collection container (45) is arranged in the forward region of the control apparatus (3).

6. Treatment device according to any preceding claim,
characterised in that,
the receiving hole (52) is arranged on the control apparatus (3).

7. Treatment device according to claim 6,
characterised in that,
the receiving hole (52) is arranged on the front side of the control apparatus (3).

8. Treatment device according to any preceding claim,
characterised in that,
there is provided a container part (43) with a supply container (44) for the flushing agent and with the collection container (45) and in that a delivery line, provided as a flexible pump hose (37), is selectively connectable with a supply container (57) for the treatment agent or with the supply container (44) for the flushing agent.

9. Method of cleaning the flexible line (7, 7a) of the medical treatment device (1) in accordance with claim 1, the line (7, 7a) being formed as a tube line for the delivery of a mineral treatment liquid from the media source (6) to the treatment instrument in the form of a handpiece (2), in which method after a treatment, or after determined or determinable time periods, a liquid flushing agent and then a gaseous medium are delivered through the line (7, 7a) and the liquid flushing agent is emptied from the tube line (7, 7a) with the gaseous medium.

## Revendications

1. Dispositif de traitement médical (1) comprenant:
- un instrument de traitement sous la forme d'une pièce à main (2) qui est reliée à un appareil de commande (3) par une conduite d'alimentation (4),
- la pièce à main (2) comprenant une partie de pièce à main antérieure et une partie de pièce à main postérieure (2a, 2b),
- une source de fluides pour une solution de traitement minérale en tant que produit de traitement, à laquelle la conduite d'alimentation (4) est connectée par l'intermédiaire d'une conduite souple (7, 7a) qui s'étend à travers elle,
- un dispositif de rinçage (20) avec une source d'agent de rinçage,
- et un dispositif de support (51) pour la pièce à main (2) sur l'appareil de commande (3),
caractérisé par le fait
- que le dispositif de rinçage (20) comporte un récipient de collecte (45) pour l'agent de rinçage usé,
- que les parties de pièce à main (2a, 2b) sont connectées l'une à l'autre par un raccord rapide,
- la conduite (7) comporte un tronçon souple (53) qui ponte la jonction (2c) entre les parties de pièce à main et est connecté de manière séparable auxdites parties de pièce à main (2a, 2b) par des raccords (54, 55)
- et que le dispositif de support (51) est agencé sous forme de trou de réception (52) recevant la partie de pièce à main postérieure (2a) et est disposé dans le voisinage du récipient de collecte (45), de telle sorte que le tronçon souple (53) puisse être suspendu dans le récipient de collecte (45).

2. Dispositif de traitement selon la revendication 1, caractérisé par le fait que le dispositif de rinçage (20) peut être connecté automatiquement, par actionnement d'éléments de commande électriques, ou manuellement à la conduite souple (7, 7a).

3. Dispositif de traitement selon la revendication 1 ou 2, caractérisé par le fait que le dispositif de rinçage (20) comprend un réservoir d'agent de rinçage (44) et une pompe à agent de rinçage (6), en particulier une pompe tubulaire.

4. Dispositif de traitement selon la revendication 3, caractérisé par le fait que la pompe à agent de rinçage (6) est une pompe commune pour l'amenée (13) du fluide de traitement et de l'agent de rinçage.

5. Dispositif de traitement selon au moins une des revendications précédentes, caractérisé par le fait que le récipient de collecte (45) est disposé dans la partie antérieure de l'appareil de commande (3).

6. Dispositif de traitement selon au moins une des revendications précédentes, caractérisé par le fait que le trou de réception (52) est disposé sur l'appareil de commande (3).

7. Dispositif de traitement selon la revendication 6, caractérisé par le fait que le trou de réception (52) est disposé sur la face avant de l'appareil de commande (3).

8. Dispositif de traitement selon au moins une des revendications précédentes, caractérisé par le fait qu'il est prévu une partie récipient (43) avec un réservoir (44) pour l'agent de rinçage et le récipient de collecte (45) et qu'une conduite d'alimentation qui se présente sous la forme d'une conduite souple de pompe (37) peut être connectée de manière sélective à un réservoir de stockage (57) pour le fluide de traitement ou au réservoir de stockage (44) pour l'agent de rinçage.

9. Procédé de nettoyage de la conduite souple (7, 7a) du dispositif de traitement médical (1) selon la revendication 1, la conduite (7, 7a) se présentant sous la forme d'un tuyau d'amenée d'un fluide de traitement minéral d'une source de fluides (6) jusqu'à l'instrument de traitement conformé en pièce à main, selon lequel après un traitement ou à intervalles de temps prédéterminés ou déterminables on envoie dans la conduite (7, 7a) un agent de rinçage liquide puis un fluide gazeux et l'agent de rinçage est éliminé de la conduite (7, 7a) par le fluide gazeux.
